# EUROPÄISCHE PATENTANMELDUNG

(11) **EP 0 754 763 A2**
(43) Veröffentlichungstag der Anmeldung: **22.01.1997**
(21) Anmeldenummer: 96110875.0
(22) Anmeldetag: 05.07.1996
(51) Int. Cl.: C12Q 1/68, C12Q 1/44, G01N 33/58, C07H 21/04

(54) **Sequenzspezifisches Nukleinsäurenachweisverfahren und Reagenz-System zu dessen Durchführung**

(30) Priorität: 14.07.1995 DE 19525632
(71) Anmelder: BAG Biologische Analysesystem GmbH, 35423 Lich (DE)
(72) Erfinder: Ellinger, Klaus, Dr., 35423 Lich (DE)
(74) Vertreter: Fuchs, Luderschmidt & Partner

(57) **Zusammenfassung**

Verfahren und Reagenz-System zum Nachweis einer spezifischen Nukleinsäuresequenz, bei dem
a) nukleinsäurehaltiges Material mit einem für die nachzuweisende Sequenz spezifischen, sowohl ein modifiziertes 3'- als auch ein modifiziertes 5'-Ende aufweisenden Oligonukleotid als Sonde hybridisiert wird, wobei wenigstens eines der beiden Enden so modifiziert ist, daß es detektierbar markiert ist;
b) das hybridisierte Material so in Spaltprodukte zerlegt wird, daß für den Fall einer Fehlstellenbildung bei der Hybridisierung 3'- und 5'-Ende der Oligonukleotidsonde voneinander getrennt werden und in verschiedenen Spaltprodukten vorliegen; und
c) die sowohl 3'- als auch 5'-Ende des Oligonukleotids aufweisenden Spaltprodukte detektiert werden.
Das Reagenz-System zur Durchführung des Verfahrens stellt zumindest die modifizierte spezifische Oligonukleotidsonde als auch ein zur speziellen Spaltung des hybridisierten Materials befähigtes Enzym bereit. Das Verfahren gestattet den sicheren Nachweis des Vorliegens einer gesuchten Nukleinsäuresequenz mit einer Auflösung bis zu einer Base.

## Beschreibung

Die Erfindung liegt auf dem Gebiet der medizinischen Diagnostik und molekulargenetischen Forschung. Insbesondere betrifft die vorliegende Erfindung ein Verfahren zum gezielten Nachweis von bestimmten Nukleinsäuresequenzen, bei dem nukleinsäurehaltiges Material mit einem für die nachzuweisende Sequenz spezifischen Oligonukleotid als Sonde hybridisiert wird; das hybridisierte Material enzymatisch in Spaltprodukte zerlegt wird; und das Vorhandensein der spezifischen Sequenz anhand der Spaltprodukte nachgewiesen wird. Ferner betrifft die Erfindung ein Reagenzien-System oder Diagnostik-Kit zur Durchführung des erfindungsgemäßen Verfahrens.

Mit steigendem Einsatz von Nachweissystemen spezifischer Nukleinsäuren im Bereich der in vitro Diagnostik sind Methoden zur sicheren und schnellen Identifizierung von Gensequenzen zunehmend gefragt. Die möglichen Gebiete der Nukleinsäurediagnostik erstrecken sich nicht nur auf den Nachweis von Krankheitserregern (Viren, Bakterien, Parasiten) sondern verstärkt auch auf die Bereiche Erbkrankheiten, Tumordiagnostik und HLA-Typisierung. Gerade hierbei und bei der Differenzierung von Erregeruntergruppen ist die Erkennung von Unterschieden in der Genomstruktur im Bereich von wenigen Basen äußerst wichtig. Durch den Einsatz neuer Methoden zur Amplifizierung von Nukleinsäuren wurden dabei bereits beachtliche Fortschritte erzielt. Die bis heute verwendeten Nachweissysteme amplifizierter und nichtamplifizierter Nukleinsäuren (Gelelektrophorese, Southern-Blot, Dot-Blot, Hybridisierung mit Oligonukleotiden) sind jedoch auf die eine oder andere Weise nachteilig. So wird entweder ein großer Zeitaufwand benötigt, der Arbeitsaufwand ist überproportional groß oder es werden aufwendige Geräte oder Apparaturen benötigt. Auch ist das Auflösungsvermögen der bekannten Verfahren noch zu verbessern. Insbesondere jedoch die Hybridisierung mit Oligonukleotiden leidet an einer relativ großen Gefahr, daß falsche positive oder negative Ergebnisse auftreten können.

Ein Hybridisierungs-Assay, bei welchem markierte Paare von Hybridisierungsreagenzien eingesetzt werden, ist Gegenstand der EP-A-0 144 914. Gemäß dieser Druckschrift werden spezifische Nukleinsäuresequenzen in einer Testprobe nachgewiesen, indem ein Hybrid zwischen der nachzuweisenden Sequenz und einer Nukleinsäuresonde mit komplementärer Sequenz gebildet wird. Dabei ist das resultierende Hybrid so ausgelegt, daß es Bindungsstellen für zwei verschiedene spezifische Bindungsreagenzien besitzt. Jedes der Bindungsreagenzien weist eine Markierung oder Label auf, wobei die Wechselwirkung zwischen den beiden Labeln ein detektierbares Signal erzeugt. Für den Fall, daß beide Label am selben Hybrid gebunden sind, unterscheidet sich das detektierbare Signal meßbar vom Signal für den Fall, daß die beiden gelabelten Reagenzien nicht an dasselbe Hybrid gebunden sind. So kann das erste Label z. B. Glucooxidase sein, während das zweite eine Peroxidase ist. Das durch die Einwirkung der Glucooxidase auf z. B. Glucose erzeugte Wasserstoffperoxid kann zum Peroxidase-Label diffundieren, um so in Gegenwart einer geeigneten Farbstoffmischung als optisches Signal detektierbar zu sein. Obwohl die geschilderte Verfahrensweise in Bezug auf die Vermeidung eines Immobilisierungsschrittes ebenso wie hinsichtlich der Vermeidung einer Trennoperation bei derartigen Verfahren durchaus von einigem Vorteil ist, besteht das grundlegende Problem, daß sich die Label nicht nur an die Bindungsreagenzien und die daran vorgesehenen Kopplungsstellen anlagern, sondern an das Hybrid direkt, was zu einer Verfälschung der Ergebnisse führt. Im ungünstigsten Fall kann es auch zu einer Anlagerung an nicht hybridisiertes Material kommen, was ein gänzlich unzutreffendes Ergebnis vortäuschen könnte.

Die EP-A-0 320 308 offenbart ein Verfahren zum gezielten Nachweis einer Nukleinsäuresequenz, bei dem das zu detektierende Nukleinsäurematerial zunächst in die Einzelstrangform überführt wird, woran anschließend vier Nukleinsäuresonden bereitgestellt werden, nämlich zwei primäre und zwei sekundäre Sonden. Die erste primäre Sonde wiederum ist ein Einzelstrang, der die Fähigkeit besitzt an ein erstes Segment eines Primärstranges der Zielnukleinsäure zu hybridisieren, während es sich bei der zweiten primären Sonde um einen Einzelstrang handelt, der an ein zweites Segment des Primärstranges der nachzuweisenden Nukleinsäure anlagern kann. Im weiteren Verlauf des Nachweises wird das 5'-Ende des ersten Segmentes des Primärstranges des Ziels relativ zum 3'-Ende des zweiten Segments des Primärstranges so positioniert, daß sich das 3'-Ende der ersten primären Sonde mit dem 5'-Ende der zweiten primären Sonde verbinden kann, wenn die Sonden an den Primärstrang des Zielnukleotids hybridisiert werden. Die dritte sekundäre Sonde ist nun in der Lage an die erste primäre Sonde zu hybridisieren und die vierte Sonde kann an die zweite primäre Sonde anlagern. Hierauf wird gemäß der EP-A-0 320 308 folgender Reaktionszyklus mehrfach wiederholt: Die Sonden in der gesamten Testprobe werden mit Nukleinsäuren hybridisiert; die hybridisierten Sonden werden zur Bildung reorganisierter verbundener Sondensequenzen gebunden; die DNA im Testansatz wird denaturiert; und die reorganisierten, verbundenen Sondensequenzen werden detektiert, wobei bei erfolgreichen Zyklen die Menge der reorganisierten verbundenen primären und sekundären Sonden zunimmt. Dieses Verfahren ist unter der Bezeichnung 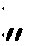 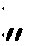 Ligase Chain Reaction" (LCR) bekannt.

Die Nukleinsäurehybridisierung und ein Amplifikationsverfahren zur Detektion von spezifischen Sequenzen, bei dem eine komplementäre, gelabelte Nukleinsäuresonde gespalten wird, sind auch aus der US-5,403,711 bekannt. Beim dort beschriebenen Verfahren wird eine doppelsträngige Target-DNA zunächst denaturiert. Zu der dann in Einzelstrangform vorliegenden Target-DNA werden ein Überschuß von gelabelten Nukleinsäuresonden (RNA) sowie Enzyme (v.a. RNaseH, die nur RNA in RNA:DNA-Hybriden spaltet) oder andere Katalysatoren zugesetzt, um die Sonden abzutrennen, die sich einmal an die Target-DNA-Sequenz angelagert hatten; die Sonden werden an den Target-DNA-Strang hybridisiert; es folgt die Spaltung der Sonden und die Rückführung der Target-DNA-Moleküle; nach mehrfacher Wiederholung der Zyklen werden die Spalt-Produkte der gelabelten Sonden gewonnen. Beim Verfahren gemäß der US-5,403,711 dient demnach die Ziel-Sequenz als Cofaktor für eine Reaktion in welcher die Sonde katalytisch gespalten wird. Nach Spaltung der Sonde bleibt die Zielsequenz intakt und kann wiederholt rezykliert werden, was im Hinblick auf das zu messende Signal zu einem relativ guten Amplifikationseffekt führt.

Einem praktischen Stand der Technik entspricht es, eine bestimmte Sequenz einer ein- oder doppelsträngigen DNA (ssDNA bzw. dsDNA) oder einer RNA nachzuweisen, indem doppelsträngiges nukleinsäurehaltiges Material (z.B. dsDNA) zunächst denaturiert wird, d. h., die Doppelstränge werden in Einzelstränge zerlegt. Bei Einzelsträngen wird üblicherweise zur Auflösung der Sekundärstruktur ebenfalls denaturiert. Danach erfolgt eine Hybridisierung, bei der ein Sondenmolekül an den Einzelstrang der DNA oder RNA angelagert wird. Als Sondenmoleküle finden hierzu Oligonukleotid-Moleküle Verwendung, die an einem Ende eine Markierung tragen, oder die nachzuweisende DNA wird mit Markierungen versehen. Als solche Markierungen im weitesten Sinne sind unter anderem radioaktive Isotope, Enzyme, Fluoreszenzfarbstoffe und dergleichen in Gebrauch.

Die Oligonukleotidsonden oder -indikatoren sind nun hinsichtlich ihrer Basensequenz so ausgelegt, daß sie sich dann an einen Einzelstrang einer DNA oder RNA anlagern, wenn dieser in einem bestimmten Abschnitt die gesuchte Nukleinsäuresequenz aufweist.

Üblicherweise ist dann eine Bindung der nachzuweisenden DNA oder der Oligonukleotidsonden an eine Festphase (Mikrotiterplatte, Nitrozellulose- oder Nylon-Filter) nötig, um nicht gebundene Markierungen abtrennen zu können. Nach deren Abtrennung lassen sich die restlichen Markierungen mit bekannten Verfahren direkt oder indirekt nachweisen.

Die Anlagerung der Oligonukleotidsonde, die sogenannte Hybridisierung kann nun überhaupt nicht, teilweise oder auch vollständig geschehen. Im ersten Fall kann bei Einhaltung der zur Hybridisierung notwendigen Reaktionsbedingungen, insbesondere im Hinblick auf Reaktionstemperatur und Salzgehalt der Reaktionsansätze, davon ausgegangen werden, daß die nachzuweisende Sequenz nicht vorhanden ist. Eine vollständige Anlagerung der Oligonukleotidsonde bedeutet, daß die gesuchte Nukleinsäuresequenz im untersuchten nukleinsäurehaltigen Material vorhanden ist.

Problematisch ist der Fall der teilweisen Anlagerung oder Hybridisierung. Es kann nämlich auch zur Anlagerung der Sonde kommen, wenn nur eine ähnliche Sequenz vorliegt. So besteht bei nicht optimalen Hybridisierungsbedingungen (pH-Wert, Salzgehalt, Temperatur) durchaus die Möglichkeit einer Anlagerung, wenn z. B. in einer einzigen oder auch mehreren Basen der Sequenz keine Bindung oder eine falsch positive Bindung zwischen Nukleotidsonde und zu untersuchendem nukleinsäurehaltigem Material erreicht wird. Dies täuscht jedoch aufgrund der weiteren Schritte des Nachweisverfahrens sowie der anschließenden Detektion fälschlicherweise das Vorhandensein der gesuchten Sequenz vor, was immer wieder Anlaß für falsche Ergebnisse und Fehldiagnosen ist.

Zum weiteren Nachweis wird nämlich gemäß dem praktischen Stand der Technik das hybridisierte Material umfassend sowohl die vollständig angelagerten Oligonukleotidsonden ebenso wie die nicht vollständig passenden Sonden, die gegebenenfalls an Sequenzen des nukleinsäurehaltigen Materials angelagert sind, die nicht mit der gesuchten Sequenz übereinstimmen, immobilisiert. Es wird also eine Bindung an eine feste Phase vorgenommen, was beispielsweise über die Markierung der Oligonukleotidsonde geschehen kann. In diesem Fall kann über eine z. B. bei der Hybridisierung weiters eingebrachte zweite Markierung ein Nachweis der gesuchten Sequenz erfolgen.

Die hierzu verwendeten Verfahren sind ebenfalls grundsätzlich bekannt. So kann die Detektion je nach Art der Markierung entweder direkt (z. B. Fluorometrie) oder indirekt (z. B. ELISA) erfolgen. Unter den indirekten Nachweismöglichkeiten sind weiterhin alle allgemein unter dem Begriff Immunoassay zusammengefaßt Testverfahren zu nennen, die auf der Spezifität immunologischer Reaktionen beruhen. Hierzu gehören neben Enzymimmunoassays auch Radioimmunoassays, Immunfluoreszenztests, Blotting-Techniken, etc.

Unabhängig vom tatsächlich angewandten Nachweissystem bleibt jedoch die Gefahr von falschen positiven oder negativen Ergebnissen, wenn es zu einer Anlagerung der Oligonukleotidsonde an die Einzelstrang DNA oder an eine RNA gekommen ist, obwohl die Sonde hinsichtlich der Basensequenz zur gesuchten Sequenz nicht vollständig komplementär ist. Wenn also eine oder mehrere Basen nicht gepaart werden konnten, es aber trotzdem zur Anlagerung der Sonde kam, wird der Nachweis einer Nukleinsäuresequenz vorgetäuscht, die überhaupt nicht im untersuchten Material vorhanden war.

Angesichts des hierin angegebenen und diskutierten Standes der Technik war es mithin Aufgabe der Erfindung, ein Verfahren der eingangs erwähnten Gattung anzugeben, das den benötigten Zeitaufwand, Arbeitsaufwand und Gerätaufwand reduziert, und das Auflösungsvermögen und die Sicherheit des Nachweises steigert. Insbesondere ist die Nachweisreaktion weniger sensitiv auf nicht-optimale Hybridisierungsparameter zu gestalten.

Dabei soll die durch die Erfindung geschaffene Nachweismethode vor allen Dingen auch mit einfachen Mitteln automatisierbar sein, um so eine einfache Routinediagnostik zu ermöglichen.

Schließlich soll auch ein einfaches Reagenz-System, bevorzugt in Form eines Diagnose-Kits zur Durchführung des Verfahrens der Erfindung bereitgestellt werden.

Gelöst werden diese sowie weitere nicht näher angegebene Aufgaben durch ein Verfahren der eingangs erwähnten Art mit den Merkmalen des kennzeichnenden Teils des Anspruches 1 sowie auch durch die in den unabhängigen nebengeordneten Ansprüchen angegebenen Maßnahmen. Vorteilhafte Modifikationen des Verfahrens der Erfindung werden in den von den unabhängigen Ansprüchen abhängigen Unteransprüchen unter Schutz gestellt. Ein Reagenz-Kit ist Gegenstand von Anspruch 21. Bevorzugte Ausführungsformen des Reagenz-System werden in den von Anspruch 21 abhängigen Ansprüchen offenbart.

Dadurch, daß bei einem Verfahren der eingangs erwähnten Art zur Hybridisierung
a') eine sowohl ein modifiziertes 3'- als auch ein modifiziertes 5'-Ende aufweisende Oligonukleotidsonde verwendet wird, wobei wenigstens eines der beiden Enden so modifiziert ist, daß es detektierbar markiert ist;
b') das hybridisierte Material so in Spaltprodukte zerlegt wird, daß für den Fall einer Fehlstellenbildung bei der Hybridisierung 3'- und 5'-Ende des Oligonukleotids voneinander getrennt werden und in verschiedenen Spaltprodukten vorliegen; und
c') die sowohl 3'- als auch 5'-Ende des Oligonukleotids aufweisenden Spaltprodukte detektiert werden;
gelingt es in nicht ohne weiteres vorhersehbarer Weise in kurzer Zeit und ohne großen Arbeits-, Geräte- oder Materialaufwand definierte Bereiche nachzuweisender Nukleinsäuren mit einem Auflösungsvermögen von bis zu einer Base zu erkennen. Durch Adaptierung der erfindungsgemäßen Methode auf Mikrotiterplatten ist es im Rahmen der Erfindung ferner möglich, die Methode größtenteils zu automatisieren, wodurch sich das Verfahren der Erfindung besonders für den Einsatz in der Routinediagnostik eignet.

Mit der Erfindung gelingt es mit hoher Zuverlässigkeit, Nukleinsäuresequenzen spezifisch in beliebigem unspezifischen nukleinsäurehaltigem Ausgangsmaterial nachzuweisen. So sind hinsichtlich der nukleinsäurehaltigen Ausgangsmaterialien keine besonderen Beschränkungen erkennbar. Es werden mit Erfolg sowohl dsDNA (doppelsträngige DNA) als auch ssDNA (einzelsträngige DNA) oder RNA analysiert.

Die zum Nachweis der gesuchten Nukleinsäuresequenzen benötigten Oligonukleotidsonden sind dem Fachmann an sich geläufig und nach herkömmlichen Verfahren erhältlich. Es handelt sich dabei um synthetisch hergestellte Nukleinsäuresequenzen (Oligonukleotide), die in einzelsträngiger, markierter Form zur Hybridisierung mit homologen oder verwandten Sequenzen führen.

Ein Kennzeichen der Erfindung besteht darin, daß sowohl am 3'-Ende als auch am 5'-Ende modifizierte Oligonukleotidsonden eingesetzt werden. Im Rahmen der Erfindung wird unter modifiziertem 3'- und 5'-Ende einer Oligonukleotidsonde verstanden, daß die terminalen Bereiche ein Label, eine Markierung oder dergleichen tragen oder daß eines der Enden an eine feste Phase gekoppelt ist.

Zur Markierung der Sonden gibt es eine Reihe von brauchbaren Verfahren. So ist zum einen eine radioaktive Markierung z. B. mit ³²P denkbar. Ferner sind auch nicht auf der radioaktiven Markierung beruhende Methoden in Gebrauch. So ist eine Kopplung mit dem relativ stabilen lichtproduzierenden Enzym Luciferase bekannt. Eine andere Möglichkeit ist die Antigenmarkierung der Sonde und Detektion über einen Antikörper-Enzym-Komplex, der eine Farbreaktion katalysiert, möglich. Schließlich sind auch Mikroautoradiographie oder Fluoreszenz möglich.

Die mit den Oligonukleotidsonden erreichte Hybridisierung der nukleinsäurehaltigen Materialien ist an sich bekannt. Unter Hybridisierung wird im Sinne der Erfindung insbesondere die Sequenzabhängige Paarung von einzelsträngigen RNA- oder DNA-Molekülen mit den Oligonukleotidsonden zu RNA/DNA-Hybriden verstanden. Die Paarung erfolgt wie beim DNA-Doppelstrang über Wasserstoffbrückenbindungen zwischen komplementären Basen. Damit ist die Hybridisierung abhängig von der Sequenzhomologie. Im Rahmen der Erfindung kann die Hybridisierung sowohl in gelöster als auch in immobilisierter Form durchgeführt werden. Hierbei kann es bei leicht unterschiedlichen Basensequenzen zu einem nicht perfekten Doppelstrang (mismatches oder Fehlpaarungen) kommen.

Ein weiterer wichtiger Aspekt der Erfindung besteht darin, daß das unter Verwendung der doppelt-modifizierten Oligonukleotidsonden erhältliche hybridisierte Material so in Spaltprodukte zerlegt wird, daß für den Fall einer Fehlstellenbildung (mismatch) bei der Hybridisierung das 3'- und 5'-Ende des Oligonukleotids voneinander getrennt werden und somit in verschiedenen Spaltprodukten vorliegen; damit ist gewährleistet, daß ein Spaltprodukt nur dann die Sonde mit beiden modifizierten Enden aufweist, wenn es vorher zu einer vollständigen Paarung mit der gesuchten Sequenz kam, d. h., im Idealfall liegt für diesen Fall kein einziges 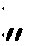 mismatch" vor.

Erfindungsgemäß werden dann genau die sowohl 3'- als auch 5'-Ende des Oligonukleotids aufweisenden Spaltprodukte detektiert, so daß eine Messung nur dann ein Signal ergibt, wenn eine 100 %ig homologe Sequenz in der zu untersuchenden Probe vorhanden war.

In bevorzugter Ausführungsform des erfindungsgemäßen Verfahrens wird zur Zerlegung des hybridisierten Materials eine einzelstrangspezifische Nuklease eingesetzt. Wenn zum Abbau des hybridisierten Materials eine einzelstrangspezifische Nuklease verwendet wird, welche bei unvollständiger Hybridisierung die Oligonukleotidsonde so abbaut, daß die beiden modifizierten Enden voneinander getrennt werden, kann daß Ziel der Erfindung besonders vorteilhaft erreicht werden. Die hierbei zum Einsatz gelangenden einzelstrangspezifischen Nukleasen sind dem Fachmann bekannt. Es kommen sowohl Endo- als auch Exonukleasen oder eine Kombination in Form einer Mischung davon in Frage. Ferner können auch Enzyme verwendet werden, die sowohl exo- als auch endospezifisch sind. Wichtig für die Erfindung ist, daß diese Nukleasen in der Lage sind, überall wo es zu einer unvollständigen Hybridisierung gekommen ist, also sog. 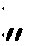 mismatches" aufgetreten sind, einen Abbau des hybridisierten Materials in Spaltprodukte oder Bruchstücke herbeizuführen, in denen die beiden Modifikationen am 3'-Ende und am 5'-Ende der Oligonukleotidsonden in verschiedenen Bruchstücken oder abgebauten Produkten vorliegen.

In besonders bevorzugter Verfahrensmodifikationen wird zur Verdau der Hybride eine einzelstrangspezifische S1-Nuklease eingestzt.

In vorteilhafter Ausgestaltung des erfindungsgemäßen Verfahrens wird zur Hybridisierung eine doppelmarkierte Oligonukleotidsonde verwendet wird, die sowohl am 3'-Ende als auch am 5'-Ende markiert ist.

Wenn Sonden mit zwei Markierungen verwendet werden, ist bevorzugt darauf zu achten, daß die Markierungen so gewählt werden, daß die Sonde über eine der beiden Markierungen an eine feste Phase gebunden oder immobilisiert werden kann, während die zweite mit einem an sich bekannten Detektionsverfahren nachweisbar sein sollte. Zweckmäßig sind daher die beiden Markierungen unterschiedlicher Natur.

Wenn eine zur Kopplung an eine feste Phase befähigte modifizierte Sonde eingesetzt wurde, ist es bevorzugt nach dem enzymatischen Verdau, die Spaltprodukte an eine feste Phase zu koppeln und die die nachzuweisende Sequenz aufweisenden Spaltprodukte von sonstigen Spaltprodukten sowie nichthybridisierten markierten Oligonukleotiden und nichthybridisiertem nukleinsäurehaltigem Material abzutrennen.

In einer Abwandlung des erfindungsgemäßen Verfahrens kann man den Nachweis auch ohne Trennung der Spaltprodukte ausführen. Wenn die beiden Markierungen so geartet sind, daß sie zur Detektion miteinander in Wechselwirkung treten müssen, kann auf eine Abtrennung der Spaltprodukte, die nicht beide Modifikationen der Oligonukleotidsonde enthalten, verzichtet werden. Mann kann in diesem speziellen Fall die Label für die Oligonukleotidsonde so auswählen, daß eine ganze Reihe von Wechselwirkungen zwischen den Markierungen für die Detektion eines positiven Signals in Frage kommen. So kann es zu chemischen, physikalischen oder elektrischen Wechselwirkungen zwischen den beiden Labeln kommen, wobei auch Kombinationen von Wechselwirkungsmechanismen denkbar sind. Eine bevorzugte Wechselwirkung zwischen den beiden Markierungen einer Oligonukleotidsonde ist z. B. die chemische Wechselwirkung. So kann eine Markierung an einer chemischen Reaktion beteiligt sein, in der ein sog. diffundierbares Mediatormolekül erzeugt wird, das dann an einer Reaktion mit der zweiten Markierung beteiligt ist, um z.B. ein detektierbares Signal zu erzeugen. Hierbei macht man sich den Effekt zu Nutze, daß die Mikroumgebung des gebundenen Hybrids eine höhere lokale Konzentration des Mediatorprodukts aufweist als die Umgebung sonstiger in der Reaktionsmischung noch enthaltener Spaltprodukte. Eine Vielzahl von Enzymen oder Katalysatoren kann in diesem Sinne erfindungsgemäß als Markierung eingesetzt werden. Beispiele umfassen u. a. Oxidoreduktasen, insbesondere solche die Nukleotide wie z.B. NAD, NADH oder ATP als Cofaktoren umfassen oder solche die Wasserstoffperoxid oder andere kleine diffundierbare Moleküle erzeugen. Zu solchen Enzymen gehören u. a. Alkoholdehydrogenasen, Glyceroldehydrogenasen, Glucose-6-phosphatdehydrogenasen, Glucooxidasen, Uricasen etc. Andere einsetzbare Klassen von Enzymen sind u. a. Hydrolasen, Transferasen, Lyasen oder Isomerasen. Bevorzugt ist für die zweite Markierung ebenfalls ein Enzym, wobei das Produkt der ersten Markierung als Substrat oder Cofaktor für die zweite Markierung dient, so daß es in der Mikroumgebung des zweiten Labels zu einer schellen Enzymreaktion kommen kann. Wenn also die erste Markierung eine Wasserstoffperoxid produzierende Oxidoreduktase ist, ist das zweite Label bevorzugt eine Peroxidase. Andere Systeme beziehen sich auf Enzyme als eine der Markierungen, die die Bildungsreaktion von prosthetischen Gruppen für ein Apo- oder Proenzym katalysieren. Selbstverständlich kommen auch nichtenzymatische Katalysatoren als Label in Frage.

Eine weitere Gruppe von Labeln betrifft den Energietransfer. Das erste Label kann eine photoemittierende Substanz sein, z. B. fluoreszierend oder lumineszierend, so daß im ersten Fall bei Bestrahlung und im zweiten Fall durch eine chemische Reaktion ein Emission erzeugt wird. Die Photoemission läßt sich z. B. durch das zweite Label absorbieren, wodurch die Emission entweder gequencht wird oder eine zweite Emission erzeugt wird, so als sei das absorbierende Label selbst eine fluoreszierende Verbindung. Einige brauchbare Fluoreszenz/Quencher-Paare sind u. a. Naphthalin/Anthracen, α-Naphthylamin/Dansyl, Tryptophan/Dansyl, Dansyl/Fluoreszein, Fluoreszein/Rhodamin, Tryptophan/Fluoreszein, N-[p-(2-Benzoxazolyl)phenyl]maleimid(BPM)/Thiochrom, BPM/8-Anilino-1-Naphthylsulfonat(ANS), Thiochrom/N-(4-Dimethylamino-3,5-dinitrophenyl)maleimid(DDPM) und ANS/DDPM. Besonders bevorzugt sind die Paare Luminol/Fluoreszein sowie Eosin/Rhodamin S.

Wie bereits erwähnt ist es für den Fall, daß eine Sonde mit zwei der vorstehend genannten Markierungen eingesetzt wird, bevorzugt, das hybridisierte Material nach einer einzelstrangspezifischen Verdau ohne Abtrennung der Spaltprodukte direkt zum Nachweis der spezifischen Sequenz zu detektieren. Isolations- oder Trennschritte können demnach in dieser Variante weitgehend entfallen.

In einer alternativen Ausführungsform dazu kann das Verfahren der Erfindung auch so durchgeführt werden, daß zur Hybridisierung eine an einem Ende detektierbar markierte und am anderen Ende an eine feste Phase gekoppelte Oligonukleotidsonde verwendet wird. Die Bindung einer der Markierungen an eine feste Phase kann also bereits vor der Hybridisierung durchgeführt werden. In diesem Fall kann in einer bevorzugten Abwandlung auch ganz auf die Bindungsmarkierung verzichtet werden. Es wird also eine bereits immobilisierte und nur einfach markierte Sonde zur Hybridisierung verwendet. In diesem Fall ist die Markierung der immobilisierten Sonde detektierbar. Bevorzugt werden nach einzelstrangspezifischem Verdau die nicht spezifischen Spaltprodukte sowie überschüssiges Ausgangsmaterial abgetrennt und die an der festen Phase gebundenen und detektierbare Markierungen aufweisenden Produkte nachgewiesen. Besonders zweckmäßig erfolgt bei dieser Variante die Abtrennung sonstiger Spaltprodukte und nichthybridisierter Ausgangsstoffe durch Waschen des an eine feste Phase gekoppelten die nachzuweisende Sequenz und wenigstens eine detektierbare Markierung aufweisenden Produkts.

Regelmäßig wird beim Verfahren der Erfindung immer dann eine Abtrennung sonstiger Spaltprodukte und nichthybridisierter Ausgangsstoffe durch Waschen des an eine feste Phase gekoppelten die nachzuweisende Sequenz und wenigstens eine detektierbare Markierung aufweisenden Produkts angestrebt, wenn eine der Modifizierungen der Oligonukleotidsonde eine feste Phase ist oder wenn eine der Modifizierungen zur Bindung an eine feste Phase ausgebildet und im Laufe des Verfahrens der Erfindung an die feste Phase gebunden wurde. Dabei ist entscheidend, daß im Zeitpunkt der Detektion der Markierung unspezifische Bruchstücke möglichst weitgehend aus dem Reaktionsansatz entfernt sind. Dies wird zweckmäßig durch geeignetes Auswaschen erreicht, wobei dann bei diesen Abwandlungen die an eine feste Phase gekoppelten und eine detektierbare Markierungen tragenden Bruchstücke nachgewiesen werden.

Demnach resultiert in vorteilhafter Abwandlung des erfindungsgemäßen Verfahrens ein Nachweis, bei dem
A) nukleinsäurehaltiges Material mit einem sowohl am 3'-Ende als auch am 5'-Ende markierten Oligonukleotid als Sonde hybridisiert wird, wobei dessen Markierung am 3'-Ende von der am 5'-Ende verschieden ist,
B) das hybridisierte Material mit einer einzelstrangspezifischen Nuklease unter Erhalt von Spaltprodukten des hybridisierten nukleinsäurehaltigen Materials abgebaut wird,
C) markierte Spaltprodukte an eine Festphase gekoppelt werden, wobei die Kopplung der markierten Spaltprodukte selektiv nur über eine der beiden verschiedenen Markierungen am 3'-Ende oder am 5'-Ende erfolgt, und
D) die Signale der anderen der beiden Markierungen zum Nachweis des Vorhandenseins der spezifischen Sequenz detektiert werden.

Alternativ hierzu wird die der Erfindung zugrundeliegende Aufgabe auch dadurch gelöst, daß
AA) nukleinsäurehaltiges Material mit einem am 3'-Ende oder am 5'-Ende markierten und am jeweiligen nicht markierten Ende an eine feste Phase gebundenen Oligonukleotid als Sonde hybridisiert wird,
BB) an die feste Phase gebundenes hybridisiertes Material mit einer einzelstrangspezifischen Nuklease abgebaut wird, CC) die erhaltenen vollständig hybridisierten an die feste
Phase gebundenen Abbauprodukte von Spaltprodukten des nicht vollständig hybridisierten nukleinsäurehaltigen Materials, von nichthybridisierten markierten Oligonukleotiden und von nichthybridisiertem nukleinsäurehaltigem Material abgetrennt werden, und
DD) die Markierungen der an die Festphase gebundenen Moleküle zum Nachweis der spezifischen Sequenz detektiert werden.

Ein weiterer Aspekt des erfindungsgemäßen Verfahrens wird schließlich in einer Vorgehensweise verwirklicht, bei der
A') nukleinsäurehaltiges Material mit einem am 3'-Ende und am 5'-Ende markierten Oligonukleotid als Sonde hybridisiert wird,
B') hybridisiertes Material mit einer einzelstrangspezifischen Nuklease abgebaut wird, und
C') die Markierungen der vollständig hybridisierten und nicht abgebauten Oligonukleotidsonde zum Nachweis der spezifischen Sequenz mittels Energietransfer detektiert werden.

Wie bereits erwähnt, ist grundsätzlich jedes nukleinsäurehaltige Material mit dem erfindungsgemäßen Verfahren analysierbar. So kann man das Verfahren der Erfindung zu einfacher Erregerdiagnostik, Erregertypisierung (Mycobakterien, Papillom-Viren etc.), zur HLA-Typisierung, in der Tumordiagnostik (Punktmutation z.B. bei p53) oder auch zum Nachweis von Erbkrankheiten ( Cystische Fibrose, Muskeldystrophie Duchene etc.) einsetzen.

Wenn das nukleinsäurehaltige Material dabei nicht in der Einzelstrangform vorliegt, kann es Problemlos denaturiert werden. Dies geschieht zweckmäßig nach der Zugabe der Oligonukleotidsonde. Bei doppelsträngigem neukleinsäurehaltigem Ausgangsmaterial kommt es dabei zur Entfaltung und Strangtrennung. Bei Einzelsträngen ist eine Denaturierung ebenfalls angezeigt, um Rückfaltungen zu trennen. Bevorzugt ist es, das nukleinsäurehaltige Material vor der Hybridisierung durch Erhöhung von Temperatur oder Verschiebung des pH-Wertes auf stark alkalische Werte, bevorzugt auf pH-Werte > 14, zu denaturieren.

Zu den erfindungsgemäß einsetzbaren Markierungen gehören u. a. grundsätzlich alle Haptene, die leicht mit ELISA-Methoden nachweisbar sind, z. B. Digoxygenin, Bromdesoxyuridin etc.); ferner alle geeigneten Fluoreszenzfarbstoffe. Unter allen möglichen Markierungen für den Nachweis der Sonde haben sich insbesondere solche als geeignet erwiesen, die auf Basis der Fluoreszenzspektrometrie nachweisbar sind. Daher ist es in besonders zweckmäßiger Ausführungsform bevorzugt, daß man als Oligonukleotidsonde ein 3'- oder 5'-Fluoresceinmarkiertes (FITC) Oligonukleotid verwendet. Dieses ist sowohl direkt als auch mittels ELISA nachweisbar.

Bevorzugt werden somit die nach Entfernung unspezifischer Spaltprodukte an der festen Phase verbliebenen FITC-Markierungen mittels direkter Fluoreszenz-Messung detektiert.

Weiterhin zweckmäßig detektiert man die an der festen Phase verbliebenen FITC-Markierungen über anti-FITC-POD mit Substrat-Umsetzung (TMB) und Photometrie (EIA).

Zur Immobilisierung der einzelstrangspezifisch abgebauten hybrdisierten doppelmarkierten Sonden-Oligonukleotide kommt jede dem Fachmann bekannte und hierfür geeignete feste Phase in Frage. Geeignet sind u. a. Mikrotiterplatten, Nitrozellulosefilter oder Nylonfilter. Bevorzugt ist es, daß man zur Immobilisierung oder Kopplung an eine feste Phase Mikrotiterplatten verwendet. Diese gestalten das erfindungsgemäße Verfahren insbesondere leicht automatisierbar.

In einem solchem Fall hat sich die Verwendung von mit Streptavidin oder Avidin beschichteten Mikrotiterplatten in Verbindung mit Biotin-markierten Oligosonden als sehr günstig erwiesen.

Gegenstand der Erfindung ist auch ein Reagenz-System, bevorzugt in Form eines Diagnose-Kits zur Durchführung des hierin vorstehend beschriebenen Verfahrens. Das Reagenz-Kit umfaßt dabei alle wesentlichen Reagenzien zur einfachen Ausführung der Erfindung. Das Reagenz-System ist in handelsüblicher Form abgepackt und enthält die nötigen Reagenzien in Form von Einzelbestandteilen oder Mischungen oder Zusammensetzungen, je nachdem welche Form möglich ist. Als Kit umfaßt es bevorzugt eine geeignete Zahl von Behältern oder Reaktionsgefäßen sowie eine Anleitung in geschriebener Form zur Durchführung des Verfahrens der Erfindung in der einen oder anderen Variante.

In jedem Fall weist das Reagenz-System zum Nachweis einer spezifischen Nukleinsäuresequenz in einer Testprobe, ein für die nachzuweisende Sequenz spezifisches, zu dieser Sequenz im wesentlichen komplementäres, in Einzelstrangform vorliegendes Oligonukleotid als Sonde auf, wobei die Sonde sowohl ein modifiziertes 3'- als auch ein modifiziertes 5'-Ende aufweist, wobei wenigstens eines der beiden Enden so modifiziert ist, daß es detektierbar markiert ist; und es weist weiterhin ein geeignetes Enzym auf, welches hybridisierte Material, das durch Anlagerung der Sonde an die gesuchte Nukleinsäuresequenz entsteht, so in Spaltprodukte zerlegt, daß für den Fall einer Fehlstellenbildung bei der Hybridisierung 3'- und 5'-Ende der Oligonukleotidsonde voneinander getrennt werden und in verschiedenen Spaltprodukten vorliegen.

In bevorzugter Ausführungsform weist das Kit als Enzym eine einzelstrangspezifische Nuklease auf. Hierbei handelt es sich äußerst zweckmäßig um eine zur Spaltung des hybridisierten Materials S1-Nuklease.

In besonders bevorzugter Ausgestaltung weist das Kit zur Hybridisierung eine doppelmarkierte Oligonukleotidsonde auf, die sowohl am 3'-Ende als auch am 5'-Ende markiert ist.

Ein der beiden Markierungen kann zweckmäßig zur Kopplung an eine feste Phase befähigt und die andere detektierbar sein.

Schließlich weist das Reagenz-System weiterhin vorteilhaft als feste Phase Mikrotiterplatten auf, an die Spaltprodukte koppelbar sind.

Besonders vorteilhaft enthält das Kit als Oligonukleotidsonde ein Fluorescein-markiertes (FITC) und mit Biotin als zur Kopplung befähigte Markierung markiertes Oligonukleotid und mit Streptavidin oder Avidin beschichtete Mikrotiterplatten als feste Phase.

Alternativ hierzu ist es auch bevorzugt, daß das Kit der Erfindung eine Oligonukleotidsonde aufweist, bei der beide Markierungen so geartet sind, daß sie zur Detektion miteinander in Wechselwirkung treten müssen.

In einer weiteren Ausführungsform ist das Kit so beschaffen, daß es eine Festphase (Mikrotiterplatte) mit markierter, gekoppelter Oligonukleotidsonde aufweist.

In weiters bevorzugter Ausgestaltung enthält ein erfindungsgemäßes Diagnose-Kit für den Nachweis gegebenenfalls amplifizierter Nukleinsäuren:
- Erreger oder Typ-spezifische Oligonukleotid-Sonde mit Doppelmarkierung (FITC/Biotin);
- Streptavidin beschichtete Mikrotiterplatte;
- Hybridisierungspuffer;
- Nuklease-Puffer;
- einzelstrangspezifische Nuklease;
- Enzymgekoppelter Antikörper;
- Enzym-Substrat;
- Waschpuffer;
- Stopp-Lösung für Enzym-Substrat-Reaktion;

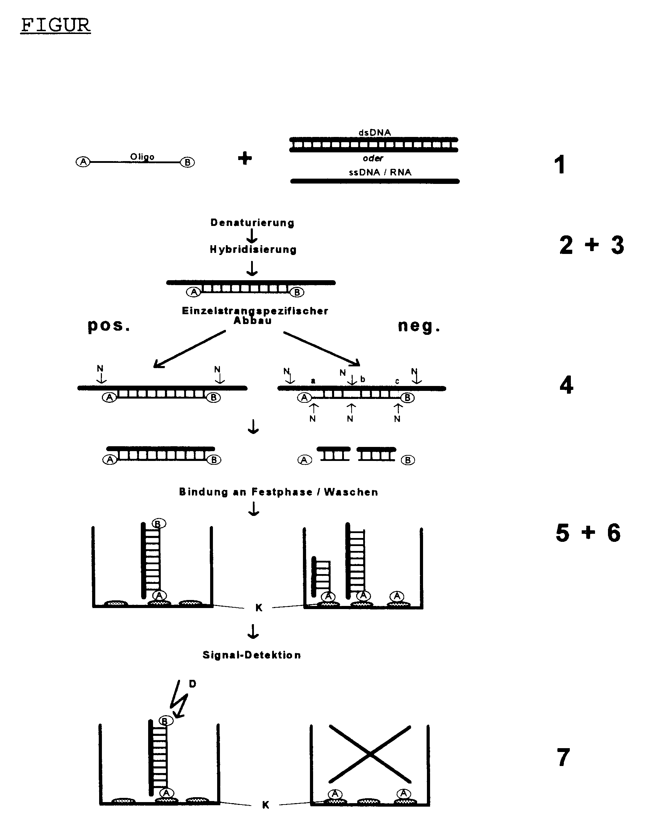

Der Gegenstand der Erfindung wird nachfolgend durch Ausführungsbeispiele und anhand der beigefügten Figur eingehender erläutert.

Die Figur zeigt eine schematische Darstellung der Einzelschritte einer bevorzugten Ausführungsform des erfindungsgemäßen Verfahrens.

In der Figur bedeutet 1 die Ausgangssubstanzen für eine bestimmte Modifikation des sequenzspezifischen Nukleinsäurenachweises. Oligo A,B steht für ein Sondenoligonukleotid mit zwei Markierungen A und B am 3'- bzw. 5'-Ende des Oligonukleotids. Die nachzuweisende Nukleinsäuresequenz kann entweder als dsDNA (doppelsträngige DNA), ssDNA (einzelsträngige DNA) oder RNA (Einzelstrangform) vorliegen.

Die Ziffern 2 + 3 bezeichnen die Schritte der Denaturierung und Hybridisierung. Man erkennt, daß im Falle einer Übereinstimmung der Sonde mit der gesuchten Sequenz eine vollständige Anlagerung der doppelmarkierten Nukleotidsonde an das nach der Denaturierung oder von vornherein als Einzelstrang vorliegende nukleinsäurehaltige Material erfolgt.

4 steht für den Schritt der einzelstrangspezifischen Abbaureaktion, welchem das hybridisierte Material aus 2 + 3 unterzogen wird. Im linken Bildteil von Schritt 4 wird die Einwirkung der einzelstrangspezifischen Nuklease N, bei der es sich um eine Endo- und/oder Exonuklease oder um eine zu beiden Reaktionen befähigte Nuklease handeln kann, auf einen vollständig hybridisierten Abschnitt des nukleinsäurehaltigen Ausgangsmaterials verdeutlicht. Man erkennt, daß die einzelstrangspezifische Nuklease die vollständig angelagerten Sonden nicht angreift. Insbesondere bleiben die beiden Markierungen A und B an einem einzigen Spaltprodukt vorhanden. Es handelt sich also um einen positiven Befund (pos.), d. h. die untersuchte DNA ist 100 % homolog zur Oligonukleotidsonde. Im rechten Bildschnitt von Schritt 4 wird ein Fall verdeutlicht, in welchem das nukleinsäurehaltige Material die gesuchte Sequenz nicht aufwies, es aber trotzdem zu einer Anlagerung der Oligonukleotidsonde kam. Man erkennt, daß in einem solchen Fall die ungepaart vorliegenden Stellen (Fehlstellen) von der einzelstrangspezifischen Nuklease angegriffen werden können, so daß es zur Bildung von Spaltprodukten kommt. Bemerkenswert hierbei ist es, daß für einen solchen Fall immer Spaltprodukte resultieren, die maximal nur eine der beiden Markierungen A oder B aufweisen.

Der Befund ist negativ (neg.), d. h. es treten Fehlpaarungen auf, die für eine Abwesenheit der gesuchten Sequenz sprechen. Mit den Buchstaben a, b und c sind in Schritt 4 die Schnittstellen für die Nukleasen bezeichnet. Endonukleasen können bei b angreifen, während Exonukleasen bei a und c reagieren.

Die Bezugszeichen 5 + 6 stehen für die Bindung der Spaltprodukte an eine feste Phase sowie die Entfernung von unspezifischen Spaltprodukten, nichthybridisertem nukleinsäurehaltigem Ausgangsmaterial und nichthybridisierten Oligonukleotidsonden. K steht in diesem Zusammenhang für ein festphasengebundenes Bindungsmolekül an welches eine der Markierungen (im gezeigten Fall A) gekoppelt werden kann. Es ist ersichtlich, daß grundsätzlich alle Spaltstücke gebunden werden, welche Markierung A aufweisen. Trotzdem unterscheiden sich die an die feste Phase gebundenen Spaltprodukte grundsätzlich nach ihrer Vorgeschichte. Wenn eine vollständige Anlagerung (Hybridisierung) der Oligonukleotidsonde stattgefunden hatte, weisen die an die feste Phase gebundenen Spaltprodukte auch eine Markierung B auf (vgl. linker Bildabschnitt des Schrittes 5 + 6). Im anderen Fall (rechter Abschnitt der Darstellung des Teilschrittes 5 + 6) kommt es zwar auch zu einer Bindung des Spaltproduktes an die feste Phase, dem gekoppelten Produkt fehlt es jedoch an der Markierung B. In Schritt 5 und 6 werden alle nicht an die feste Phase gebundenen Spaltprodukte sowie nicht umgesetzte Edukte durch geeignete Maßnahmen aus dem Analysen-System entfernt, z. B. durch dem Fachmann geläufige Waschmethoden.

Mit dem Bezugszeichen 7 wird schließlich der Detektionsschritt bezeichnet. D ist dabei ein für die Markierung B spezifisches Nachweissystem, wie z. B. Fluorometrie oder ELISA. Es wird im Detektionsschritt 7 demnach nur ein Signal erhalten, wenn ein nukleinsäurehaltiges Material untersucht wurde, welches die zur Oligonukleotidsonde 100 % homologe Sequenz aufwies.

Das beschriebene Prinzip wurde in Testversuchen erprobt. In Modellversuchen werden ein Oligonukleotid, welches eine bekannte zu detektierende Nukleinsäuresequenz enthält, oder eine amplifizierte DNA, welche die gesuchte Sequenz aufweist, mit einer unspezifischen Tracer-DNA 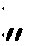 verdünnt". Dieses nukleinsäurehaltige Material, welches die bestimmte zu analysierende Nukleinsäuresequenz in Form des Oligonukleotids oder der amplifizierten DNA mit Gewißheit aufweist, wird zusammen mit einem doppelmarkierten Oligonukleotid, welches als Sonde speziell auf die nachzuweisende Nukleinsäuresequenz anspricht, denaturiert und anschließend einer einzelstrangspezifischen Nuklease-Reaktion unterzogen. Anschließend wird das in der Nuklease-Reaktion erhaltene Reaktionsvolumen an eine feste Phase gebunden, nichtgebundene Sonden sowie unspezifische Spaltprodukte werden entfernt und die an die Festphase gebundenen Sonden werden mittels ELISA gemessen.

Für die einzelnen Schritte werden folgender Reaktionsansatz sowie die angegebene allgemeine Verfahrensweise eingehalten:
A)Allgemeiner Reaktionsansatz für Denaturierung und Hybridisierung:

| | |
|---|---|
| - Doppelmarkiertes Oligonukleotid | 1 pmol |
| - NaCl | 100 mM |
| - zu detektierende Nukleinsäure | |
| Oligonukleotid | 1 pmol |
| oder | |
| amplifizierte DNA | 5-40 µl (= ca. 0,1 - 1 µg) |

B)Denaturierung
Zur Denaturierung wird ein Reaktionsansatz für ca. 5 Minuten auf etwa 95 °C erhitzt.
C)Hybridisierung
Anschließend wird er schnell auf ca. 15 °C abgekühlt und dann ca. 2 Min. bei dieser Temperatur gehalten. Hierbei findet die Hybridisierung statt.
D)Verdünnung sowie einzelstrangspezifische Nuklease-Reaktion
Zu einem Reaktionsansatz wird Tracer DNA (unspezifische DNA, z.B. aus Heringssperma)in einer Menge von ca. 1 µg zugegeben. Das Gesamtvolumen des Ansatzes beträgt ca. 100 µl.
Ein denaturierter, hybridisierter und derart verdünnter Reaktionsansatz wird mit etwa 10 Units einer einzelstrangspezifischen Nuklease (S1-Nuklease der Firma Amersham) versetzt.
Die Nuklease-Reaktion wird in einem vom jeweiligen Enzym abhängigen enzymspezifischen Puffer durchgeführt. Für die genannte S1-Nuklease wurden 30 mM Natriumacetat, 280 mM NaCl und 1 mM ZnSO₄ verwendet. Hierbei werden die Reaktionsansätze in der Regel ca. 30 Min. bei einer Temperatur von ca. 37 °C gehalten. Die Reaktion wird durch Zugabe von EDTA, welches die für die Funktion der S1-Nuklease notwendigen Zn-Ionen abfängt, abgebrochen. Das Abbruchmittel wird dabei in einer solchen Menge zugegeben, daß seine Menge im Reaktionsansatz bei beendigter Zugabe mindestens 5 mM beträgt.
E)Bindung an die Festphase
Das gesamte Reaktionsvolumen wird auf Streptavidinbeschichtete Mikrotiterplatten gegeben und dann 5 Min. bei Raumtemperatur inkubiert.
F)Entfernung unspezifischer Spaltprodukte und nichtgebundener Sonden
Die inkubierten Mikrotiterplatten werden fünfmal mit einer Mischung aus PBS (phosphate buffered saline) und 0,01 TWEEN® (Type: 20) bei Raumtemperatur gewaschen, wobei nicht an die Mikrotiterplatten gebundene Produkte aus dem Reaktionsansatz entfernt werden.
G)Bindung von enzym-markierten Antikörpern (antiFluorescein, Peroxidase gekoppelt)
Antikörperverdünnung nach Herstellervorschrift (hier Antikörper Boehringer, Verdünnung 1:2500)
Zugabe von je 100 µl in jede Vertiefung der Mikrotiterplatte. Daraufhin wird die Mikrotiterplatte luftdicht abgeklebt und bei 37 °C ca. 30 Min. inkubiert.
H)Entfernung nicht gebundener Antikörper
Die inkubierten Mikrotiterplatten werden fünfmal mit einer Mischung aus PBS (phosphate buffered saline) und 0,01 TWEEN® (Type: 20) bei Raumtemperatur gewaschen, wobei nicht gebundene Antikörper aus dem Reaktionsansatz entfernt werden.
I)Enzym-Substrat-Reaktion
Zu den ausgewaschenen Mikrotiterplatten setzt man 100 µl TMB (Tetramethylbenzidin) zu und inkubiert ca. 10 Min. in Dunkelheit bei Raumtemperatur. Die Reaktion wird durch Zugabe von 100 µl 1N Schwefelsäure abgestoppt.
J)Messung
Mit einem Photometer (ELISA-Reader) wird die Absorption bei 450 nm gemessen.

### Beispiel 1

Gemäß den vorstehend angegebenen allgemeinen Verfahrensschritten A) bis J) wurden verschiedene Oligonukleotide als zu detektierende DNA mit einer Oligonukleotidsonde getestet. Als Sonde wurde ein am 5'- und am 3'-Ende markiertes Oligonukleotid folgender Sequenz verwendet:

### Sonde S1:

Biotin GGT CTT CCT GGA CCT CGT CTC Fitc

Als zu detektierende Oligonukleotide wurden Testsubstanzen mit bekannter Sequenz eingesetzt:

### Oligonukleotid O1:

CAG CCA GAA GGA CCT GGA GCA GAG GCG

Erlaubt eine vollständige Anlagerung der Sonde S1, ist also 100 % homolog zu S1.

### Oligonukleotid O2:

CAG CCA GAA GGA A*CT GGA GCA GAG GCG

Damit weicht O2 in einer Base (1 interne Fehlpaarung) von der zur Sonde S1 100 %ig homologen Sequenz ab, besitzt also ein internes 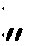 mismatch"

### Oligonukleotid O3:

CAG CCA GAA GGA CCT GGA GCA GAA* GCG

Damit weicht O3 in einer Base (1 Fehlpaarung am 3'-Ende,) von der Sonde S1 ab, besitzt also ein externes 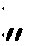 mismatch" bezüglich der Sonde S1.

### Oligonukleotid O4:

CAG T*CA GAA GGA CCT GGA GCA GAG GCG

Damit weicht O4 in einer Base (1 Fehlpaarung am 5'-Ende,) von der Sonde S1 ab, besitzt also ein externes 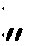 mismatch" bezüglich der Sonde S1.

Wie aus den angegebenen Sequenzen ersichtlich ist, waren die Testoligomeren (O1 bis O4) in allen Fällen an beiden Enden um jeweils 3 Basen verlängert, um teilweise Realbedingungen (längere Amplifikate) zu imitieren.

Gemessen wurden die optischen Dichten bei 450 nm (OD₄₅₀) für die 4 Testansätze mit den Oligonukleotiden O1 - O4, wobei die S1-Reaktion (einzelstrangspezifische Nuklease-Reaktion) jeweils in 310 und in 410 mM NaCl durchgeführt wurde, sowie für Kontrollversuche K1 und K2. Im Kontrollversuch K1 wurde die Sonde S1 eingesetzt, wobei jedoch die S1-Reaktion ausgelassen wurde. Im Kontrollversuch K2 wurde keine Sonde eingesetzt.
Die Ergebnisse der Untersuchungen sind in der nachfolgenden Tabelle 1 zusammengefaßt:

**Tabelle 1**

| Test | OD₄₅₀x1000 S1-Reaktion in 310 mM Na⁺ | OD₄₅₀ x1000 S1-Reaktion in 410 mM Na⁺ | OD₄₅₀ x1000 |
|---|---|---|---|
| O1 | 1262 | 3311 | |
| O2 | 937 | 3282 | |
| O3 | 193 | 779 | |
| O4 | 1041 | 2824 | |
| K1*) | | | >3500 |
| K2**)) | | | 108 |

| | | | |
|---|---|---|---|
| *) = positive Kontrolle (Sonde ohne S1-Reaktion) | | | |
| **)= negative Kontrolle (ohne Sonde) | | | |

Man erkennt, daß die übereinstimmende Sequenz (O1) sowohl bei Durchführung der S1-Reaktion in 0 mM NaCl als auch bei Durchführung der S1-Reaktion in 100 mM NaCl signifikant größere OD₄₅₀-Werte ergibt, als die abweichenden Oligonukleotide O2 bis O4. Erstaunlich ist insbesondere, daß verglichen mit einem nur eine einzige interne Abweichung in einer Base aufweisenden Oligonukleotid (O2) bei Durchführung der S1-Reaktion ohne NaCl eine deutliche Differenz im OD₄₅₀-Wert resultiert. Dies zeigt das hohe Auflösungsvermögen bis zu einer Base der erfindungsgemäßen Nachweismethode.

### Beispiel 2

Gemäß den obigen allgemeinen Verfahrensschritten A) bis J) wurden verschiedene amplifizierte DNA's, die die zu detektierende Nukleinsäuresequenz vollständig oder mit geringer Abweichung beinhalten mit einer Oligonukleotidsonde getestet. Als Sonde wurde das ebenfalls in Beispiel 1 eingesetzte am 5'- und am 3'-Ende markierte Oligonukleotid folgender Sequenz verwendet:

### Sonde S1:

Biotin GGT CTT CCT GGA CCT CGT CTC Fitc

Die Sonde S1 ist spezifisch, d.h. 100 % homolog zu HLA-DRB1*0101

Als zu detektierende Amplifikate wurden amplifizierte DNA's aus Patienten mit unterschiedlichen HLA-DR-Typen eingesetzt. Es handelte sich dabei im einzelnen um folgende Testsubstanzen:

### Amplifikat A1:

DRB1*0101

A1 enthält die mit der Sonde S1 nachzuweisende Basensequenz zu 100%.

### Amplifikat A2:

DRB1*0103

A2 weist verglichen mit der durch die Sonde S1 nachzuweisenden Sequenz interne und externe Fehlpaarungen auf.

### Amplifikat A3:

DRB1*0301

A3 weist verglichen mit der durch die Sonde S1

### nachzuweisenden Sequenz nur interne Fehlpaarungen auf. Amplifikat A4:

DRB1*16

A4 weist verglichen mit der durch die Sonde S1 nachzuweisenden Sequenz nur externe Fehlpaarungen auf.

Bei allen 4 Testamplifikaten wurde jeweils die gesamte HLA-DR-Region, ausgehend von verschiedenen Patienten-DNA's, amplifiziert.

Bei den Ansätzen wurden jeweils verschiedene Mengen Amplifikat ( 5-40 µl ) untersucht. Die Konzentrationen der eingesetzten Amplifikate wurden nach Messung auf ca. 100 ng/µl eingestellt. Die S1-Reaktion (einzelstrangspezifische Nuklease-Reaktion) wurde jeweils in 150 mM NaCl mit 5 Units Enzym bei 37 °C für 30 Min. ausgeführt. Gemessenen wurden die optischen Dichten bei 450 nm (OD₄₅₀) für die 4 Testansätze mit den Amplifikaten A1 - A4. In Kontrollversuchen wurde die Sonde S1 eingesetzt, wobei jedoch die S1-Reaktion ausgelassen wurde (K3) bzw. es wurde keine Sonde eingesetzt (K4).

Die Ergebnisse der Untersuchungen sind in der nachfolgenden Tabelle 2 zusammengefaßt:

**Tabelle 2**

| Test | OD₄₅₀x1000 40 µl Amplifikat | OD₄₅₀ x1000 20 µl Amplifikat | OD₄₅₀ x1000 10 µl Amplifikat | OD₄₅₀ x1000 5 µl Amplifikat | OD₄₅₀ x1000 |
|---|---|---|---|---|---|
| A1 | 3117 | 3108 | 2657 | 2283 | |
| A2 | 353 | 288 | 220 | 190 | |
| A3 | 344 | 256 | 267 | 176 | |
| A4 | 2235 | 1727 | 1138 | 738 | |
| K3^{*)} | | | | | >3500 |
| K4^{**)}⁾ | | | | | 159 |

| | | | | | |
|---|---|---|---|---|---|
| *) = positive Kontrolle (Sonde ohne S1-Reaktion) | | | | | |
| **)= negative Kontrolle (ohne Sonde) | | | | | |

Aus den in der Tabelle 2 angegebenen Werten ist ersichtlich, daß die zu 100 % zur eingesetzten Oligonukleotidsonde passende amplifizierte DNA im größten OD₄₅₀-Wert resultierte. Gleich ob die amplifizierte DNA interne und/oder externe Fehlpaarungen aufwies, in jedem Fall wurden kleinere OD₄₅₀-Wert erhalten, verglichen mit dem Amplifikat (A1), das die mit der eingesetzten Oligonukleotidsonde nachzuweisende Sequenz in 100%iger Übereinstimmung aufwies. Selbst im Vergleich zu einem nur externe Fehlpaarungen aufweisenden Amplifikat (A4) sind die erhaltenen Unterschiede im OD₄₅₀-Wert signifikant und lassen somit eine Auflösung im Bereich von einer Base einer Nukleinsäuresequenz zu.

### Beispiel 3

Gemäß den vorstehend angegebenen allgemeinen Verfahrensschritten A) bis J) wurden wie in Beispiel 1 dieselben Oligonukleotide O1 bis O4 als zu detektierende DNA mit der Sonde S1 getestet. In einem Teil des Tests wurden anstelle von 10 Units der S1-Nuklease jeweils 2,5 Units einer einzelstrangspezifischen Mungbean-Nuklease (Endo-Nuklease) eingesetzt. Weitere Einzelheiten sowie die gemessenen Ergebnisse sind in der nachfolgenden Tabelle 3 zusammengefaßt:

**Tabelle 3**

| | 10 Units S1-Nuklease | | 2,5 Units Mungbean |
|---|---|---|---|
| Test | OD₄₅₀x1000 S1-Reaktion in 360 mM Na⁺ | OD₄₅₀ x1000 S1-Reaktion in 410 mM Na⁺ | OD₄₅₀ x1000 Reaktion in 230 mM Na⁺ |
| O1 | 1361 | 2295 | 1807 |
| O2 | 821 | 1962 | 1304 |
| O3 | 442 | 201 | 696 |
| O4 | 735 | 1088 | 645 |

Der vorstehenden Tabelle ist entnehmbar, daß auch unter Verwendung von Mungbean-Nuklease beim Oligonukleotid O1 verglichen mit den Ologonukleotiden O2 bis O4 der höchste OD₄₅₀-Wert resultiert. Ferner signalisieren die Differenzen zwischen den OD₄₅₀-Werten bei den verschiedenen Testoligonukleotiden eine Auflösung von bis zu einer einzigen Base Abweichung, besonders beim Vorhandensein eines externen 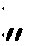 mismatches" (vgl. O3 unter Einsatz der S1-Nuklease).

### Beispiel 4

Gemäß den vorstehend angegebenen allgemeinen Verfahrensschritten A) bis J) wurden wie in Beispiel 2 dieselben Amplifikate A1 bis A4 als zu detektierende DNA mit der Sonde S1 getestet. Es wurden bei jeweils 3 Wiederholungen 5 Units S1-Nuklease bei der einzelstrangspezifischen Verdau eingesetzt. Die weiteren Versuchsbedingungen sowie die Ergebnisse sind in Tabelle 4 angegeben:

**Tabelle 4**

| Test | OD₄₅₀ x1000 eingesetzte Menge je 30 µl Amplifikat 410 mM Na⁺ | | |
|---|---|---|---|
| A1 | 1086 | 1249 | 1089 |
| A2 | 86 | 267 | 194 |
| A3 | 94 | 473 | 91 |
| A4 | 538 | 259 | 713 |

Weitere Vorteile und Ausführungsformen der Erfindung ergeben sich aus den nachfolgenden Patentansprüchen.

## Patentansprüche

1. Verfahren zum Nachweis einer spezifischen Nukleinsäuresequenz, bei dem
a) nukleinsäurehaltiges Material mit einem für die nachzuweisende Sequenz spezifischen Oligonukleotid als Sonde hybridisert wird;
b) das hybridisierte Material enzymatisch in Spaltprodukte zerlegt wird; und
c) das Vorhandensein der spezifische Sequenz anhand der Spaltprodukte nachgewiesen wird,
**dadurch gekennzeichnet**, **daß**
a') eine sowohl ein modifiziertes 3'- als auch ein modifiziertes 5'-Ende aufweisende Oligonukleotidsonde verwendet wird, wobei wenigstens eines der beiden Enden so modifiziert ist, daß es detektierbar markiert ist;
b') das hybridisierte Material so in Spaltprodukte zerlegt wird, daß für den Fall einer Fehlstellenbildung bei der Hybridisierung 3'- und 5'-Ende des Oligonukleotids voneinander getrennt werden und in verschiedenen Spaltprodukten vorliegen; und
c') die sowohl 3'- als auch 5'-Ende des Oligonukleotids aufweisenden Spaltprodukte detektiert werden.

2. Verfahren nach Anspruch 1,
**dadurch gekennzeichnet**, **daß**
das hybridisierte Material zur Zerlegung in Spaltprodukte mit einer einzelstrangspezifischen Nuklease behandelt wird.

3. Verfahren nach Anspruch 1 oder 2,
**dadurch gekennzeichnet**, **daß**
zur Spaltung des hybridisierten Materials eine S1-Nuklease verwendet wird.

4. Verfahren nach einem oder mehreren der vorhergehenden Ansprüche,
**dadurch gekennzeichnet**, **daß**
zur Hybridisierung eine doppelmarkierte Oligonukleotidsonde verwendet wird, die sowohl am 3'-Ende als auch am 5'-Ende markiert ist.

5. Verfahren nach Anspruch 4,
**dadurch gekennzeichnet**, **daß**
eine Oligonukleotidsonde verwendet wird, bei denen eine der beiden Markierungen zur Kopplung an eine feste Phase befähigt und die andere detektierbar ist.

6. Verfahren nach Anspruch 5,
**dadurch gekennzeichnet**, **daß**
die Spaltprodukte an eine feste Phase gekoppelt werden und die nachzuweisende Sequenz aufweisende Spaltprodukte von sonstigen Spaltprodukten sowie nichthybridisierten markierten Oligonukleotiden und nichthybridisiertem nukleinsäurehaltigem Material abgetrennt werden.

7. Verfahren nach Anspruch 6,
**dadurch gekennzeichnet**, **daß**
die Abtrennung sonstiger Spaltprodukte und nichthybridisierter Ausgangsstoffe durch Waschen des an eine feste Phase gekoppelten die nachzuweisende Sequenz und wenigstens eine detektierbare Markierung aufweisenden Produkts erfolgt.

8. Verfahren nach einem oder mehreren der vorhergehenden Ansprüche,
**dadurch gekennzeichnet**, **daß**
man als Oligonukleotidsonde ein Fluorescein-markiertes (FITC) Oligonukleotid verwendet.

9. Verfahren nach Anspruch 8,
**dadurch gekennzeichnet**, **daß**
man die an der festen Phase verbliebenen FITC-Markierungen mittels direkter Fluoreszenz-Messung detektiert.

10. Verfahren nach Anspruch 8,
**dadurch gekennzeichnet**, **daß**
man die an der festen Phase verbliebenen FITC-Markierungen über anti-FITC-POD mit Substrat-Umsetzung (TMB) und Photometrie (EIA) detektiert.

11. Verfahren nach einem oder mehreren der vorhergehenden Ansprüche,
**dadurch gekennzeichnet**, **daß**
man zur Immobilisierung oder Kopplung an eine feste Phase Mikrotiterplatten verwendet.

12. Verfahren nach Anspruch 11,
**dadurch gekennzeichnet**, **daß**
man Biotin als zur Kopplung befähigte Markierung und mit Streptavidin oder Avidin beschichtete Mikrotiterplatten als feste Phase verwendet.

13. Verfahren nach Anspruch 4,
**dadurch gekennzeichnet**, **daß**
beide Markierungen so geartet sind, daß sie zur Detektion miteinander in Wechselwirkung treten müssen.

14. Verfahren nach Anspruch 13,
**dadurch gekennzeichnet**, **daß**
das hybridisierte Material nach einem einzelstrangspezifischen Verdau ohne Abtrennung der Spaltprodukte direkt zum Nachweis der spezifischen Sequenz detektiert wird.

15. Verfahren nach einem oder mehreren der Ansprüche 1 bis 3,
**dadurch gekennzeichnet**, **daß**
zur Hybridisierung eine an einem Ende detektierbar markierte und am anderen Ende an eine feste Phase gekoppelte Oligonukleotidsonde verwendet wird.

16. Verfahren nach Anspruch 15,
**dadurch gekennzeichnet**, **daß**
nach einzelstrangspezifischem Verdau die nicht spezifischen Spaltprodukte sowie überschüssiges Ausgangsmaterial abgetrennt wird und die an der festen Phase gebundenen und detektierbare Markierungen aufweisenden Produkte nachgewiesen werden.

17. Verfahren zum Nachweis einer spezifischen Nukleinsäuresequenz,
**dadurch gekennzeichnet**, **daß**
A) nukleinsäurehaltiges Material mit einem sowohl am 3'-Ende als auch am 5'-Ende markierten Oligonukleotid als Sonde hybridisiert wird, wobei dessen Markierung am 3'-Ende von der am 5'-Ende verschieden ist;
B) das hybridisierte Material mit einer einzelstrangspezifischen Nuklease unter Erhalt von Spaltprodukten des hybridisierten nukleinsäurehaltigen Materials abgebaut wird;
C) markierte Spaltprodukte an eine Festphase gekoppelt werden, wobei die Kopplung der markierten Spaltprodukte selektiv nur über eine der beiden verschiedenen Markierungen am 3'-Ende oder am 5'-Ende erfolgt, und
D) die Signale der anderen der beiden Markierungen zum Nachweis des Vorhandenseins der spezifischen Sequenz detektiert werden.

18. Verfahren zum Nachweis einer spezifischen Nukleinsäuresequenz,
**dadurch gekennzeichnet**, **daß**
AA) nukleinsäurehaltiges Material mit einem am 3'-Ende oder am 5'-Ende markierten und am jeweiligen nicht markierten Ende an eine feste Phase gebundenen Oligonukleotid als Sonde hybridisiert wird,
BB) an die feste Phase gebundenes hybridisiertes Material mit einer einzelstrangspezifischen Nuklease abgebaut wird,
CC) die erhaltenen vollständig hybridisierten an die feste Phase gebundenen Abbauprodukte von Spaltprodukten des nicht vollständig hybridisierten nukleinsäurehaltigen Materials, von nichthybridisierten markierten Oligonukleotiden und von nichthybridisiertem nukleinsäurehaltigem Material abgetrennt werden, und
DD) die Markierungen der an die Festphase gebundenen Moleküle zum Nachweis der spezifischen Sequenz detektiert werden.

19. Verfahren zum Nachweis einer spezifischen Nukleinsäuresequenz,
**dadurch gekennzeichnet**, **daß**
A') nukleinsäurehaltiges Material mit einem am 3'-Ende und am 5'-Ende markierten Oligonukleotid als Sonde hybridisiert wird,
B') hybridisiertes Material mit einer einzelstrangspezifischen Nuklease abgebaut wird, und
C') die Markierungen der vollständig hybridisierten und nicht abgebauten Oligonukleotidsonde zum Nachweis der spezifischen Sequenz mittels Energietransfer detektiert werden.

20. Verfahren nach wenigstens einem der vorhergehenden Ansprüche,
**dadurch gekennzeichnet**, **daß**
das nukleinsäurehaltige Material vor der Hybridisierung durch Erhöhung von Temperatur oder Verschiebung des pH-Wertes auf etwa 14 denaturiert wird.

21. Reagenz-System zum Nachweis einer spezifischen Nukleinsäuresequenz in einer Testprobe, aufweisend ein für die nachzuweisende Sequenz spezifisches, zu dieser Sequenz im wesentlichen komplementäres, in Einzelstrangform vorliegendes Oligonukleotid als Sonde,
**dadurch gekennzeichnet**,
i) daß die Sonde sowohl ein modifiziertes 3'- als auch ein modifiziertes 5'-Ende aufweist, wobei wenigstens eines der beiden Enden so modifiziert ist, daß es detektierbar markiert ist; und
ii) daß es weiterhin ein geeignetes Enzym aufweist, welches hybridisiertes Material, das durch Anlagerung der Sonde an die gesuchte Nukleinsäuresequenz entsteht, so in Spaltprodukte zerlegt, daß für den Fall einer Fehlstellenbildung bei der Hybridisierung 3'- und 5'-Ende der Oligonukleotidsonde voneinander getrennt werden und in verschiedenen Spaltprodukten vorliegen.

22. Reagenz-System nach Anspruch 21,
**dadurch gekennzeichnet**, daß
es als Enzym eine einzelstrangspezifische Nuklease aufweist.

23. Reagenz-System nach Anspruch 22,
**dadurch gekennzeichnet**, **daß**
es zur Spaltung des hybridisierten Materials eine S1-Nuklease aufweist.

24. Reagenz-System nach einem oder mehreren der Ansprüche 21 bis 23,
**dadurch gekennzeichnet**, **daß**
es zur Hybridisierung eine doppelmarkierte Oligonukleotidsonde aufweist, die sowohl am 3'-Ende als auch am 5'-Ende markiert ist.

25. Reagenz-System nach Anspruch 24,
**dadurch gekennzeichnet**, **daß**
es eine Oligonukleotidsonde aufweist, bei der eine der beiden Markierungen zur Kopplung an eine feste Phase befähigt und die andere detektierbar ist.

26. Reagenz-System nach Anspruch 25,
**dadurch gekennzeichnet**, **daß**
es weiterhin als feste Phase Mikrotiterplatten aufweist, an die Spaltprodukte koppelbar sind.

27. Reagenz-System nach einem oder mehreren der Ansprüche 21 bis 26,
**dadurch gekennzeichnet**, **daß**
es als Oligonukleotidsonde ein Fluorescein-markiertes (FITC) Oligonukleotid aufweist.

28. Reagenz-System nach Anspruch 25,
**dadurch gekennzeichnet**, **daß**
es ein mit Biotin als zur Kopplung befähigte Markierung markiertes Oligonukleotid und mit Streptavidin oder Avidin beschichtete Mikrotiterplatten als feste Phase aufweist.

29. Reagenz-System nach Anspruch 24,
**dadurch gekennzeichnet**, **daß**
es eine Oligonukleotidsonde aufweist, bei der beide Markierungen so geartet sind, daß sie zur Detektion miteinander in Wechselwirkung treten müssen.

30. Reagenz-System nach einem oder mehreren der Ansprüche 21 bis 23,
**dadurch gekennzeichnet**, **daß**
es zur Hybridisierung eine an einem Ende detektierbar markierte und am anderen Ende an eine feste Phase gekoppelte Oligonukleotidsonde aufweist.
